# EUROPEAN PATENT APPLICATION

(11) **EP 2 236 129 A2**
(43) Date of publication of application: **06.10.2010**
(21) Application number: 10159177.4
(22) Date of filing: 06.04.2010
(51) Int. Cl.: A61K 9/127

(54) **Liposomal ALA Pharmaceutical and Cosmeceutical Compositions and Methods of Treatment**

(30) Priority: 02.04.2009 US 166131 P
(71) Applicant: Sesvalia USA, LLC, Davie FL 33331 (US)
(72) Inventor: Sanmiguel, Gabriel Serrano, Atlanta, GA 30324 (US); Zamora, Joaquin Melendez, Valencia 46130 (ES)
(74) Representative: Harrison Goddard Foote

(57) **Abstract**

Liposomal ALA pharmaceutical and cosmeceutical compositions, and uses of same are disclosed herein. In an embodiment, a Liposomal ALA composition of the present disclosure is used in for photodynamic therapy. In an embodiment, a photodynamic therapy system is disclosed that includes nanocorpuscles comprising 5-aminolevulinic acid in a scattering agent, wherein a concentration of the 5-aminolevulinic acid is from about 0.01% to about 10%; and a light source for activating the 5-aminolevulinic acid. In an embodiment, a photodynamic therapy method is disclosed that includes administering nanocorpuscles comprising 5-aminolevulinic acid to a target tissue in a patient; and activating the 5-aminolevulinic acid with a light source. In an embodiment, a kit is disclosed.

## Description

### FIELD

The embodiments disclosed herein relate to compositions useful for therapeutic or cosmetic purposes, e.g. cosmeceutical compositions, and more particularly to liposomal 5-aminolevulinic acid compositions and methods of using the compositions.

### BACKGROUND

Photodynamic therapy (PDT) involves the use of photochemical reactions mediated through the interaction of photosensitizing agents, light, and oxygen for the treatment of malignant or benign diseases. Conventional PDT is a 2-step procedure. In the first step, a photosensitizer is administered to a patient by one of several routes (e.g., topical, oral, intravenous), and the photosensitizer is allowed to be taken up by target cells. The second step involves the activation of the photosensitizer in the presence of oxygen with a specific wavelength of light directed toward the target tissue. Because the photosensitizer is preferentially absorbed by hyperproliferative tissue and the light source is directly targeted on the lesional tissue, PDT achieves dual selectivity, minimizing damage to adjacent healthy structures.

### SUMMARY

Liposomal ALA pharmaceutical and cosmeceutical composition and methods of treatment are disclosed herein.

In one aspect the invention provides a photodynamic therapy system comprising: a composition comprising nanocorpuscles comprising 5-aminolevulinic acid in a scattering agent; and a light source for activating the 5-aminolevulinic acid.

The nanocorpuscles may comprise at least one lipid bilayer, the lipid bilayer comprising at least one phospholipid and at least one tensoactive agent or derivatives thereof.

The the scattering agent may be water.

The system may further comprise nanocorpuscles comprising a flavonoid, optionally wherein the flavonoid is phloretin. The nanocorpuscles of the system may therefore comprise, or consist of, nanocorpuscles comprising phloretin or another flavonoid.

The system may further comprise nanocorpuscles comprising a metal chelate. The nanocorpuscles of the system may therefore comprise, or consist of, nanocorpuscles comprising a metal chelate. The metal chelate may be an iron chelate, optionally wherein the iron chelate is an iron-chelating zinc salt, e.g. selected from zinc chloride, zince gluconate or zinc acetate or a combination thereof, or is an iron-chelating copper salt, e.g. copper gluconate or copper acetate, or a combination thereof, or is a combination of such zinc and copper salts. As the metal chelate may be mentioned a source of metal-complexing agent, e.g. iron-complexing agent.

In embodiments, an output wavelength of the light source is in the visible spectrum.

Also provided by the invention is a kit for photodynamic therapy comprising:
a unit dose of a photosensitizer composition, the photosensitizer composition comprising nanocorpuscles comprising 5-aminolevulinic acid in a scattering agent;
a unit dose of a penetration enhancer product, the penetration enhancer product comprising nanocorpuscles comprising a flavonoid in a solution;
a unit dose of a chelating product composition the chelating product comprising nanocorpuscles comprising a metal chelate in a solution; and
a unit dose of an oxygen product, the oxygen product comprising oxygen saturated liposomes and 10 mg/ml of fructose 1, 6 bisphosphate encapsulated in unilamellar liposomes.

In embodiments, including without limitation embodiments of the kit, the nanocorpuscles further comprise one or both of a flavonoid, e.g. phloretin, and a metal chelate and particularly an iron chelate. The iron chelate may be an iron-chelating zinc salt, e.g. selected from zinc chloride, zinc gluconate or zinc acetate, or a combination thereof, or may be an iron-chelating copper salt, e.g. copper gluconate or copper acetate, or a combination thereof, or may be a combination of such zinc and copper salts. As the metal chelate may be mentioned a source of metal-complexing agent, e.g. iron-complexing agent.

In a further embodiment there is provided a composition comprising nanocorpuscles comprising 5-aminolevulinic acid, optionally in a scattering agent, for therapeutic use. The composition is optionally for use in the treatment of acne vulgaris by photodynamic therapy or for use in the treatment of photoaging of the skin by photodynamic therapy.

In some embodiments, the scattering agent is water. In some embodiments, the nanocorpuscles comprise, e.g. consist of, nanocorpuscles comprising a flavonoid, optionally wherein the flavonoid is phloretin. In some embodiments, the nanocorpuscles comprise, e.g. consist of, nanocorpuscles comprising an iron chelate. The iron chelate may be an iron-chelating zinc salt, e.g. selected from zinc chloride, zinc gluconate or zinc acetate, or a combination thereof, or may be an iron-chelating copper salt, e.g. copper gluconate or copper acetate, or a combination thereof, or may be a combination of such zinc and copper salts. As the metal chelate may be mentioned a source of metal-complexing agent, e.g. iron-complexing agent.

The composition is optionally for use in treating a condition selected from lung cancer, gastrointestinal tract cancers, esophageal cancer, peritoneal cancer, colorectal cancer, tumors of the small intestine, cancers of the anus, pleura cancer, brain cancer, eye cancer, skin cancer, atherosclerosis, infectious diseases, rheumatoid arthritis, cutaneous t-cell lymphoma, Kaposi's sarcoma, malignant melanoma, carcinoma in situ, keratoacanthoma, extramammary paget's disease, melasma, viral warts, acne vulgaris, vitiligo, lichen sclerosus and atrophicus, sebaceous gland hyperplasia, Psoriasis, hidradenitis suppurativa, actinic keratosis, psoriasis vulgaris, verrucae vulgaris, molluscum contagiosum, actinic chelitis, Hirsutism, alopecia areata, bowen disease, stretch marks, skin damage, skin photoaging, and combinations thereof.

The invention includes a method for photodynamic treatment to improve the appearance of the skin comprising:
administering nanocorpuscles comprising 5-aminolevulinic acid to the skin of a subject;
   and
activating the 5-aminolevulinic acid with a light source.

In some embodiments, the nanocorpuscles comprise, e.g. consist of, nanocorpuscles comprising a flavonoid, optionally wherein the flavonoid is phloretin. In some embodiments, the nanocorpuscles comprise, e.g. consist of, nanocorpuscles comprising an iron chelate. The iron chelate may be an iron-chelating zinc salt, e.g. selected from zinc chloride, zinc gluconate or zinc acetate, or a combination thereof, or may be an iron-chelating copper salt, e.g. copper gluconate or copper acetate, or a combination thereof, or may be a combination of such zinc and copper salts. As the metal chelate may be mentioned a source of metal-complexing agent, e.g. iron-complexing agent.

In embodiments of the subject matters of the invention, the nanocorpuscles comprise at least one lipid bilayer, the lipid bilayer comprising at least one phospholipid, e.g. phosphatidyl chlorine, and at least one tensoactive agent, optionally wherein the tensoactive agent (e.g. detergent or surfactant) is a bile salt or another tensoactive agent described herein.

In certain embodiments of the subject matters of the invention, 5-aminolevulinic acid is in an amount of from 0.01% to 10%, the percentage being weight %/volume of the composition comprising the nanocorpuscles, and optionally is in an amount of from 0.01% to 5%, e.g. 0.1% to 10%, 0.1% to 5% or 0.1% to 4%, for example 0.5% to 10%, 0.5% to 5% or 0.5% to 4%, as in the case of 2.5% to 10% or 2.5% to 5%. Illustratively, the amount of 5-aminolevulinic acid expressed in this way may be from 1% to 5%, e.g. 1% to 4%, as in the case of 1% to 2.5%. In certain other embodiments of the subject matters of the invention, 5-aminolevulinic acid is in an amount of from 0.01% to 10%, the percentage being weight %/volume of the nanocorpuscles, and optionally is in an amount of from 0.01% to 5%, e.g. 0.1% to 10%, 0.1% to 5% or 0.1% to 4%, for example 0.5% to 10%, 0.5% to 5% or 0.5% to 4%, as in the case of 2.5% to 10% or 2.5% to 5%. Illustratively, the amount of 5-aminolevulinic acid expressed in this way is from 1% to 5%, e.g. 1% to 4%, as in the case of 1% to 2.5%.

According to aspects illustrated herein, a photodynamic therapy system includes nanocorpuscles comprising 5-aminolevulinic acid in a scattering agent, wherein a concentration of the 5-aminolevulinic acid is from about 0.01% to about 10%; and a light source for activating the 5-aminolevulinic acid.

According to aspects illustrated herein, a kit for photodynamic therapy includes a unit dose of a photosensitizer composition, the photosensitizer composition comprising nanocorpuscles comprising the photosensitizer 5-aminolevulinic acid in a scattering agent; a unit dose of a penetration enhancer product, the penetration enhancer product comprising nanocorpuscles comprising a flavonoid in a solution; a unit dose of a chelating product, the chelating product comprising nanocorpuscles comprising a metal chelate in a solution; and a unit dose of an oxygen product, the oxygen product comprising oxygen saturated liposomes and 10 mg/ml of fructose 1, 6 bisphosphate encapsulated in unilamellar liposomes.

According to aspects illustrated herein, a photodynamic therapy method includes administering nanocorpuscles comprising 5-aminolevulinic acid to a target tissue in a patient; and activating the 5-aminolevulinic acid with a light source.

According to aspects illustrated herein, nanocorpuscles comprising 5-aminolevulinic acid in a scattering agent can be used for the preparation of a medicament for the treatment of acne vulgaris by photodynamic therapy (PDT).

According to aspects illustrated herein, nanocorpuscles comprising 5-aminolevulinic acid in a scattering agent can be used for the preparation of a medicament for the treatment of photoaging of the skin by photodynamic therapy (PDT).

According to aspects illustrated herein, nanocorpuscles comprising 5-aminolevulinic acid can be used in the manufacture of a photosensitizer for use in photodynamic therapy treatment of acne vulgaris.

According to aspects illustrated herein, nanocorpuscles comprising 5-aminolevulinic acid can be used in the manufacture of a photosensitizer for use in photodynamic therapy treatment of photoaging of the skin.

### BRIEF DESCRIPTION OF THE DRAWINGS

The presently disclosed embodiments will be further explained with reference to the attached drawings, wherein like structures are referred to by like numerals throughout the several views. The drawings shown are not necessarily to scale, with emphasis instead generally being placed upon illustrating the principles of the presently disclosed embodiments.
**FIG. 1** is a schematic illustration summarizing the biosynthesis of porphyrins, the pathway responsible for the conversion of 5-aminolevulinic acid ("ALA") to hemoglobin. The scheme includes an illustration of the enzymatic reactions converting ALA to protoporphyrin IX.
**FIG. 2** is a schematic illustration of an embodiment of a Liposomal ALA composition of the present disclosure prior to administration into target tissue.
**FIG. 3** is a schematic illustration showing the Liposomal ALA composition of **FIG. 2** after absorbed into target tissue, and the conversion of ALA into protoporphyrin IX.
**FIG. 4** is a schematic illustration showing the generation of reactive oxygen species (ROS) formed from a photochemical reaction activated by directing light of appropriate wavelengths onto the protoporphyrin IX shown in **FIG. 3****.**
**FIG. 5** is a graph showing the depth penetration of light into skin at various wavelengths.
**FIG. 6A** shows a patient's face with visible sun damage and solar keratoses.
**FIG. 6B** shows a close-up of the patient's forehead from **FIG. 6A**. The close-up shows that this patient has suffered a phototoxic reaction.
**FIG. 6C** shows the patient in **FIG. 6A** and **FIG. 6B** after a single ALA photodynamic therapy treatment of the present disclosure.
**FIG. 7A** shows a patient's face with noticeable inflammatory acne.
**FIG. 7B** shows improvement of the patient's face shown in **FIG. 7A** after four treatments with ALA photodynamic therapy of the present disclosure at one month interval.

While the above-identified drawings set forth presently disclosed embodiments, other embodiments are also contemplated, as noted in the discussion. This disclosure presents illustrative embodiments by way of representation and not limitation. Numerous other modifications and embodiments can be devised by those skilled in the art which fall within the scope and spirit of the principles of the presently disclosed embodiments.

### DETAILED DESCRIPTION

As used herein, the term "scattering agent" refers to a watery solution which comprises water. In an embodiment, the scattering agent further comprises electrolytes such as salts (for example, NaCl, buffers). In an embodiment, the scattering agent is water.

As used herein, the term "nanocorpuscles" includes nanoparticles, micellar elements, substances that may be converted into micelles (reversible), liposomes (uni-, oligo- or multilamellar) and nanocolloids, with a mean diameter lower than 500 nm, preferably lower than 250 nm, and most preferably lower than 200 nm, with standard deviations around 30%. In an embodiment, the nanocorpuscles have a mean diameter ranging from about 100 nm to about 150 nm. In an embodiment, the liposomal lipid is a phosphatidylcholine (PC). In an embodiment, the PC comprises from about 12% to about 17% palmitic acid, from about 2% to about 5% stearic acid, from about 12% to about 15% oleic acid, from about 60% to about 70% linoleic acid and from about 3% to about 7% linolenic acid.

As used herein, the terms "ALA composition", "ALA nanocorpuscle composition", and "Liposomal ALA composition" are used interchangeably to refer to compositions of the present disclosure including the active ingredient D-Aminolevulinic acid (dALA, δ-ALA, or 5-aminolevulinic acid hereinafter "ALA"), or pharmaceutically acceptable equivalents thereof, encapsulated in nanocorpuscles. In some embodiments, other ingredients are present in the liposomes of a Liposomal ALA composition of the present disclosure including, but not limited to, gel formers, membrane stabilizers, preservatives, antioxidants, salts, buffering substances, flavenoids, trace metals, proteins and vitamins. A Liposomal ALA composition of the present disclosure can be formulated as a cream, an ointment, a solution, a serum, a lacquer spray, a mist spray, a skin stick, a paste, a pledget, a wipe, a cleanser, a gel or suitable combinations thereof. In an embodiment, the Liposomal ALA composition of the present disclosure is formulated as a chemical solution. In an embodiment, the Liposomal ALA composition of the present disclosure is formulated as a mist or a nano-spray.

As used herein, the terms "cosmeceutical composition" or "photosensitizer composition" refers to a composition that comprises a Liposomal ALA composition as described above alone or a Liposomal ALA composition as described above and at least one additional product. In an embodiment, the additional product can be formulated as a cream, an ointment, a solution, a serum, a lacquer spray, a mist spray, a skin stick, a paste, a pledget, a wipe, a cleanser, a gel or suitable combinations thereof. In an embodiment, the additional product used in a cosmeceutical composition of the present disclosure is formulated as a chemical solution. In some embodiments, a cosmeceutical composition of the present disclosure comprises a Liposomal ALA composition and at least one of an oxygen product, a penetration enhancer product, and a chelating product, including, but not limited to, a liposomal zinc salt product, a liposomal lactoferrin-peroxidase product, a liposomal copper product, a liposomal mix of zinc and copper (or its salts) product, a liposomal zinc chloride, copper (ZnCl-CuSO₄) and lactoferrin-peroxidase mix product. In an embodiment, a cosmeceutical composition of the present disclosure may help to minimize unwanted side effects during photodynamic therapy treatment. In an embodiment, a cosmeceutical composition of the present disclosure is sufficiently designed to rapidly diffuse into target tissue. In such embodiments, an ALA nanocorpuscle composition of a cosmecuetical composition of the present disclosure may allow the ALA to rapidly diffuse into the skin, at a rate that is faster then that of conventional ALA. In an embodiment, an ALA nanocorpuscle composition of a cosmeceutical composition of the present disclosure may speed up the treatment time for a photodynamic therapy by allowing the ALA to penetrate the skin quickly and allowing the ALA to diffuse rapidly to target tissue so that protoporphyrin IX may accumulate in the target tissue.

As used herein, the term "unit dose" is intended to mean an effective amount of substance, or an effective number of articles, adequate for a single session. By way of example, a unit dose of a composition of the present disclosure for the face may be defined as enough solution for a person to coat the face. The surface area of the face may vary somewhat from person to person. Thus, a person using a unit dose may have excess solution left over. It is desirable to provide enough solution that even the above-average sized face will have an effective amount of coverage. In an embodiment, a unit dose of a solution of the present disclosure is contained within a container. In an embodiment, a unit dose of a solution of the present disclosure is contained in an ampoule.

Photodynamic therapy (PDT) involves the use of photochemical reactions mediated through the interaction of photosensitizing agents, light, and oxygen for the treatment of malignant or benign diseases. Conventional PDT treatment is a 2-step procedure. In the first step, a photosensitizer is administered to a patient by one of several routes (e.g., topical, oral, intravenous), and the photosensitizer is allowed to be taken up by target cells (which can take from about 3 hours to about 8 hours). Once sufficient levels of the photosensitizer accumulate within the target tissue, the photosensitizer can be activated in the presence of oxygen by applying or directing selected wavelengths of light toward the target tissue. Because the photosensitizer is preferentially absorbed by hyperproliferative tissue and the light source is directly targeted on the lesional tissue, PDT can achieve dual selectivity, minimizing damage to adjacent healthy structures.

### D-Aminolevulinic acid ("ALA")

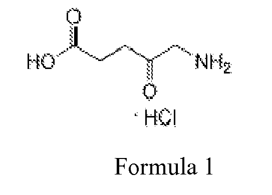

D-Aminolevulinic acid (dALA or δ-ALA or 5-aminolevulinic acid hereinafter "ALA") (Formula 1), can be used as a photosensitizer in PDT. Currently, the only FDA-approved indication for ALA photodynamic therapy (PDT) in dermatology is for the treatment of AKs. Common off-label uses include the treatment of basal cell carcinoma (BCC), photoaging, acne vulgaris, and Bowen disease. ALA is the first compound in the porphyrin synthesis pathway, the pathway that leads to hemoglobin formation in mammals. ALA is a prodrug of protoporphyrin IX. It is believed that a majority of cells of the human body can transform ALA into porphyrins, although accumulation of porphyrin may differ between various tissues and cell types. Application of ALA to human skin may lead to porphyrin accumulation in sebaceous glands and the epidermis. Neoplastic cells may accumulate more porphyrins than normal cells. As a result, photodynamic therapy using ALA may be beneficial for the treatment of actinic keratoses (AKs), Bowen disease, basal cell carcinoma (BCC), photoaging, and acne vulgaris, to name a few.

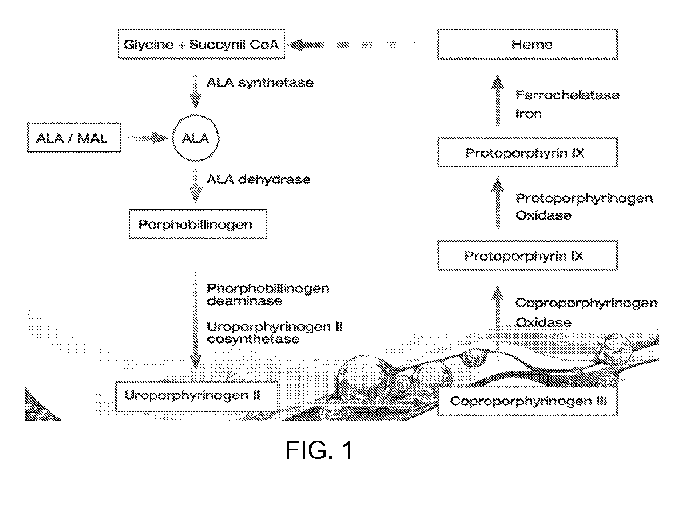

**FIG. 1** is a schematic showing the porphyrin synthesis pathway responsible for the conversion of 5-aminolevulinic acid ("ALA") to hemoglobin, including an illustration of the enzymatic reactions converting ALA to protoporphyrin IX. As shown in **FIG. 1****,** ALA's role in the porphyrin synthesis pathway allows ALA to function as a prodrug of protoporphyrin IX. ALA is a hydrophilic molecule, making it difficult to bypass the natural skin barrier. As a result, relatively high concentrations of conventional ALA (up to 20%) may be required to achieve significant levels of accumulation of protoporphyrin IX within target tissue. Conventional ALA also requires a relatively long incubation period. For instance, conventional ALA may require as long as from about 3 hours to about 8 hours of contact time with the skin to achieve complete transformation, and maximum accumulation of, protoporphyrin IX inside target tissues. It is believed that topical application of conventional ALA induces a photosensibilization of lesional tissue through its transformation to protoporphyrin IX inside the target tissue. Once the lesional tissue has been photosensibilized by sufficient accumulation of protoporphyrin IX in the tissue, the target tissue may be exposed to light of an appropriate wavelength. In most instances, conventional ALA applications apply an occlusive dressing to the skin to avoid the possibility of light inducing a reaction before ALA reaches the target tissue. Occlusive dressings have also been used to allow for greater penetration of conventional ALA into the skin. During the irradiation step, pain may be significant when using conventional ALA, for example, erythema, swelling and burning sensations are frequently observed.

Conventional ALA is typically available in a prepackaged plastic tube containing two sealed glass ampules, one with the ALA powder and the other with the hydroalcoholic solution (Levulan). The vehicle ampule contains 1.5 mL of solution composed of ethanol (48% v/v), water, laureth-4, isopropyl alcohol, and polyethylene glycol. The other ampule contains 354 mg of ALA hydrochloric acid as a dry powder. The applicator tube is enclosed in a protective cardboard sleeve with a cotton applicator. Due to the relative short shelf life (hours or days), the topical solution (final ALA concentration of 20%) of conventional ALA must be prepared just prior to application. Due to the instability of the ALA molecule in water solutions, at neutral and basic pHs, the ALA degradation is high. The molecular instability of conventional ALA may be seen by a color change in the solution which changes from a transparent color to a yellowish color as the ALA molecule degrades in solution. Conversely, at acidic pHs, stability of the ALA molecule increases. The pH in conventional ALA products currently marketed is adjusted near neutrality, and at this pH ALA is in salt form, however, the active form of ALA needed to activate the porphyrin synthesis pathway is the acid. As a result, the pH of the product should be near to its pkₐ, (e.g., pH of about 4) to be effective. In addition to ALA, methylaminolevulinate (MAL) is another conventional PDT photosensitizer. MAL is more lipophilic than ALA, and is also transformed to ALA once inside the skin.

In an embodiment, cosmeceutical compositions, kits and methods are provided for use in photodynamic therapy (PDT). In an embodiment, cosmeceutical compositions, kits and methods are provided for use in photodynamic therapy (PDT) to treat a variety of medical conditions, diseases and disorders, as well as dermatological applications, benign and malignant. In an embodiment, cosmeceutical compositions of the present disclosure can be used for the treatment of conditions including, but not limited to, lung cancer, gastrointestinal tract cancers including: esophageal cancer, tumors of the stomach (peritoneal cancer), colorectal cancer, tumors of the small intestine, cancers of the anus, pleura cancer, brain cancer, eye cancer, skin cancer (basocellular carcinoma), atherosclerosis, infectious diseases, rheumatoid arthritis, cutaneous T-cell lymphoma, Kaposi's sarcoma, malignant melanoma, carcinoma in situ (CIS), keratoacanthoma, extramammary Paget's disease, melasma, viral warts, acne vulgaris, vitiligo, lichen sclerosus and atrophicus, sebaceous gland hyperplasia, Psoriasis, hidradenitis suppurativa, Actinic keratosis, Psoriasis vulgaris, verrucae vulgaris, molluscum contagiosum, Actinic chelitis, Hirsutism, alopecia areata, Bowen disease, stretch marks, enhance healing (e.g., skin healing), skin photorejuvenation/photoaging, and many other medical conditions affecting lungs, brain, esophagus.

The products of the invention may be used for therapeutic purposes, for example to treat the medical conditions mentioned in the preceding paragraph. The products of the invention may be used for cosmetic purposes, in particular to improve the appearance of the skin, for example in the treatment of stretch marks and the cosmetic aspects of skin aging and photoaging. Cosmetic treatments may include, by way of example, treatment of one or more of wrinkling, skin thinning, hyperpigmentation and dyschromia.

In an embodiment, a Liposomal ALA composition of the present disclosure comprises ALA encapsulated in nanocorpuscles. In an embodiment, a Liposomal ALA composition of the present disclosure comprises a scattering of ALA nanocorpuscles in a watery solution as a scattering agent. In an embodiment, a concentration of ALA in the nanocorpuscles is from about 0.001% to about 10% wt%/volume of the composition. In an embodiment, a concentration of ALA in the nanocorpuscles is from about 0.010% to about 5% wt%/volume of the composition. In an embodiment, a concentration of ALA in the nanocorpuscles is from about 0.10% to about 4% wt%/volume of the composition. In an embodiment, a concentration of ALA in the nanocorpuscles is about 0.5%. In an embodiment, a concentration of ALA in the composition is about 1.0%. In an embodiment, a concentration of ALA in the composition is about 2.5%. In an embodiment, a concentration of ALA in the composition is about 5.0%.

In another embodiment, a concentration of ALA in the nanocorpuscles is from about 0.001% to about 10% wt%/volume of the nanocorpuscles. In an embodiment, a concentration of ALA in the nanocorpuscles is from about 0.010% to about 5% wt%/volume of the nanocorpuscles. In an embodiment, a concentration of ALA in the nanocorpuscles is from about 0.10% to about 4% wt%/volume of the nanocorpuscles. In an embodiment, a concentration of ALA in the nanocorpuscles is about 0.5%. In an embodiment, a concentration of ALA in the nanocorpuscles is about 1.0%. In an embodiment, a concentration of ALA in the nanocorpuscles is about 2.5%. In an embodiment, a concentration of ALA in the nanocorpuscles is about 5.0%.

Without wishing to be bound to any one theory, it is believed that a Liposomal ALA composition of the present disclosure comprising a low concentration of ALA can be beneficial for treatment therapies. For example, concentrations as low as about 0.5% to about 1% may provide clinical results and at the same time may reduce side effects. It is believed that encapsulating ALA in nanocorpuscles allows a significant decrease of the dose compared to conventional ALA. Lower doses of ALA may result decreased side effects and increased tolerance and safety. Due to the nature of the lipid based nanocorpuscles (e.g., natural skin ingredients), skin reactions resulting from ALA encapsulated in nanocorpuscles may be mild, if not absent. In an embodiment, the ALA compositions of the present disclosure produce a "barrier effect" to the skin, opening the skin membrane to the compositions. By way of illustration, and not of limitation, a patient applying the ALA compositions of the present disclosure may experience a pinkish color, however, the reaction is not painful.

In an embodiment, the nanocorpuscles comprise at least one phospholipid and at least one tensoactive agent. In an embodiment, the nanocorpuscle phospholipid is a phosphatidylcholine (PC). In an embodiment, the nanocorpuscles of a Liposomal ALA composition is present in a co-solvent or co-scattering agent. In an embodiment, the co-solvent is a pharmaceutically acceptable alcohol. In an embodiment, the pharmaceutically acceptable alcohol is ethanol. In an embodiment, the pH of a Liposomal ALA composition of the present disclosure is about 6. In an embodiment, the liposomes have a diameter of about 100 nm. In an embodiment, the pH of a Liposomal ALA composition ranges from between about 1 to about 10. In an embodiment, the pH of a Liposomal ALA composition ranges between about 2 to about 8. In an embodiment, the pH of a Liposomal ALA composition ranges from about 2.5 to about 5.

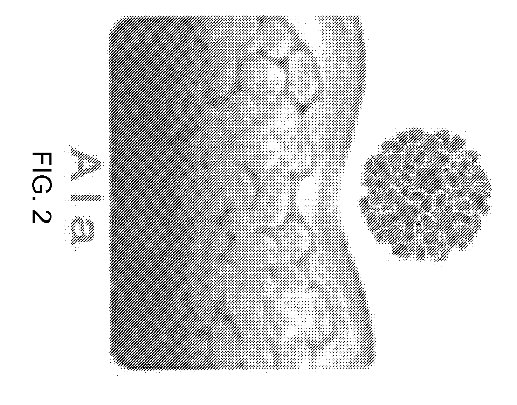

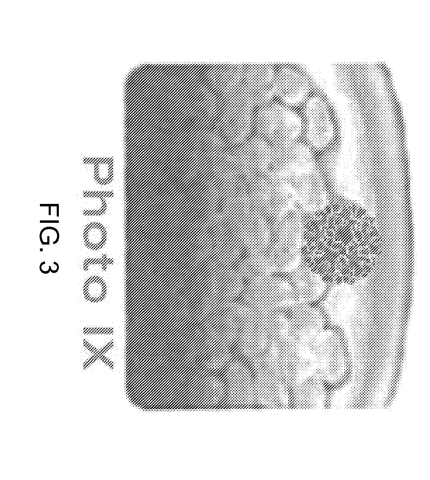

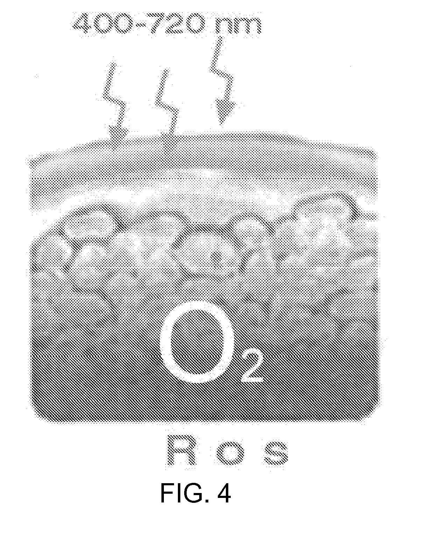

Without wishing to be bound to any one theory, it is believed that the disadvantages seen in conventional ALA products can be overcome by encapsulating ALA into nanocorpuscles as described herein. **FIG. 2** is a schematic illustration of an embodiment of a Liposomal ALA composition of the present disclosure prior to administration to target tissue in the skin. In the embodiment shown in **FIG. 2**, the Liposomal ALA composition is administered topically, although other routes of administration are possible, including, but not limited to, oral administration and parenteral administration. In an embodiment, ALA encapsulated in nanocorpuscles are believed to have superior physicochemical characteristics that allow the encapsulated ALA to rapidly diffuse into target tissues. As illustrated in the schematic of **FIG. 3****,** ALA is converted to protoporphyrin IX inside the skin. **FIG. 4** shows protoporphyrin IX being activated by light of the appropriate wavelength during a PDT treatment. In an embodiment, applying or directing a light of an appropriate wavelength toward target tissue may trigger a photochemical reaction transforming protoporphyrin IX into reactive oxygen species. In an embodiment, directing or applying a light of an appropriate wavelength onto the skin may trigger a photochemical reaction transforming protoporphyrin IX into reactive oxygen species. In an embodiment, an appropriate wavelength of light comprises a wavelength corresponding to a peak of the porphyrin excitation spectrum. It is believed that applying a wavelength of light corresponding to the peak of the porphyrin excitation spectrum in tissues may be used to efficiently generate a therapeutic effect. Notably, the Soret band (approximately 405-420 nm) is the most important excitation peak of protoporphyrin IX and is included in the spectral output of the US Food and Drug Administration (FDA)-approved Blu-U device, which is used with conventional ALA. As illustrated in **FIG. 4**, by directing light of appropriate wavelengths inside the skin, a photodynamic reaction develops inside the skin leading to the generation of reactive oxygen species (ROS). In an embodiment, ALA encapsulated in nanocorpuscles allows a significant decrease in dosage compared to conventional ALA. Notably, ALA encapsulated in nanocorpuscles may be effective in doses having concentrations of ALA as low as 0.5% whereas conventional ALA typically requires concentrations as high as 20% to be effective. It is believed that providing lower dose ALA results in decreased side effects and increased safety. In an embodiment, encapsulating ALA in nanocorpuscles transforms conventional ALA from a substantially hydrophilic molecule to an amphiphilic compound.

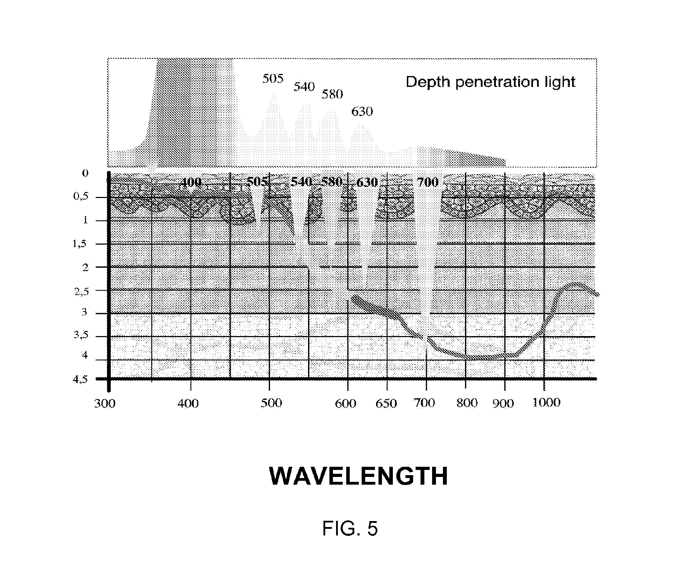

**FIG. 5** shows the skin depth penetration of light of various wavelengths. Without wishing to be bound by any one theory, it is believed that the depth penetration of light into the skin may correlate to the efficacy of a PDT treatment. That is, it is believed that longer output wavelengths of light may correspond to increased depth penetration absorbed light. As shown in **FIG. 5**, the Soret band, about 405 nm to about 420 nm may effectively allow depth penetration sufficient to generate a therapeutic effect for a PDT treatment. As further illustrated in **FIG. 5****,** light having wavelengths as much as 700 nm can effectively penetrate target tissue. In an embodiment, a light source providing various wavelengths can be useful for generating a therapeutic effect with a PDT treatment. In an embodiment, a light source providing output wavelengths in the visible spectrum may generate a therapeutic effect during a PDT treatment. In an embodiment, light having an output wavelength in the Soret band (about 405 nm to about 420 nm) efficiently generates a therapeutic effect in a patient. As shown in **FIG. 5**, the depth penetration and absorption light of at about 630 nm may be optimal for generating a therapeutic effect in target tissue. As shown in **FIG. 5**, light penetration at 630 nm corresponds well with substantial penetration into the dermis.

Following blue or red light activation, porphyrins are excited to a higher energy triplet state, which can either emit light (fluorescence) or generate reactive oxygen species, such as singlet oxygen or free radicals. The generation of singlet oxygen species, labeled type 2 photochemical reactions, are believed to predominate in PDT. Because singlet oxygen does not travel very far within a cell, the molecular effects are influenced by the intracellular localization of the photosensitizer at the time of light exposure. Porphyrins derived from ALA are mainly localized in the vicinity of mitochondria, which can lead to apoptosis or necrosis of malignant cells upon light exposure. Both phenomena have been shown to be induced following ALA PDT

In an embodiment, an ALA nanocorpuscle composition of a cosmeceutical composition of the present disclosure comprises unilamellar liposomes. In an embodiment, an ALA nanocorpuscle composition of a cosmeceutical composition of the present disclosure comprises multilamellar liposomes. In an embodiment, an ALA nanocorpuscle composition of a cosmeceutical composition of the present disclosure comprises oligolamellar liposomes. In an embodiment, an ALA nanocorpuscle composition of a cosmeceutical composition of the present disclosure includes at least one lipid bilayer comprising at least one phospholipid and at least one tensoactive agent (e.g., a detergent or surfactant) or a derivative of the tensoactive agent. In such embodiments, the phospholipid used in the ALA nanocorpuscle composition comprises an amphiphilic substance having a hydrophilic polar head and an apolar lipophilic tail. In an embodiment, the amphiphilic substance comprises lipids. In an embodiment, the amphiphilic substance comprises lipoids. While not wishing to be bound to any one theory, it is believed that the use of amphiphilic substances can lead to faster skin penetration and provide a deep, homogenous distribution within target sites. In an embodiment, the lipids comprise physiologically tolerant lipids. It is believed that the use of physiologically tolerant natural lipids may reduce undesirable side effects (e.g., inflammation) and also may lead to enhanced penetration and diffusion into the skin tissue. In an embodiment, the lipids comprise skin-compatible lipids. In an embodiment, the lipids can be used to moisturize the skin. In an embodiment, physiologically tolerant natural lipids include, but are not limited to, steroids, phospholipids, sphingolipids and glycolipids may be beneficial. Examples of suitable lipids include, but are not limited to, cerebrosides and ceramides, natural phosphocolines, natural phosphatidic or phosphatidylglycerol acid, as well as in this case lysophospholipids, fatty acids and their derivatives. In an embodiment, the liposomal lipid is a phosphatidylcholine (PC).

In an embodiment, a Liposomal ALA composition of the present disclosure comprises unilamellar liposomes, about 50 mg/ml PC, about 5 mg/ml of 5-ALA in an aqueous phase. In an embodiment, the concentration of the phospholipid PC is between about 0.3% and about 20%.

In an embodiment, the tensoactive agent is added to the lipids that form the lipid membrane of a ALA nanocorpuscle composition of the present disclosure in order enhance the nanocorpuscles penetration into the skin and mucous membranes. Tensoactive agents may be selected, in part, based on physiological tolerance to the tensoactive agent. In an embodiment, the tensoactive agent comprises a biological substance. In such embodiments, the biological substances include, but are not limited to, bile acids and derivatives of bile acids, glycosides, maltese, and thioglycosides such as lysophospholipids. In an embodiment, the tensoactive agent is a bile salt selected from the group consisting of sodium cholate, sodium desoxycholate, sodium glycocholate, sodium taurocholate, sodium taurodeoxycholate, sodium ursocholate and sodium quenoxycholate. In an embodiment, the tensoactive agent is a polysorbate or derivative, polyoxyethylene, PEG-10 cetyl ether and PEG-10 sterile ether at concentrations ranging between about 0.01% to about 30%.

In an embodiment, a Liposomal ALA composition of the present disclosure is produced by combining phospholipids with tensoactive and mixing until obtaining a homogeneous solution. In a separate vessel, ALA may be dissolved in water or in a combination of water and another water soluble solvent, such us alcohol and glycols, this solution of ALA is incorporated to the first mix of phospholipids with the tensoactive agent. The resulting mix is subjected to the application of mechanical energy, especially by vibration or agitation, and the solution is sterilized by filtration to obtain nanocorpuscles of ALA. If desired, a pH regulator can be added. In such embodiments, pH regulators include, but are not limited to, sodium hydroxide, potassium hydroxide, ethanolamine and derivatives, citric acid, lactic acid, and other similar substances capable of regulating pH.

In an embodiment, the ratio of alcohol/phospholipid ranges between about 0.5/1 and about 3/1. In an embodiment, the ratio of alcohol/phospholipid is 1/1, in volume/weight. In such embodiments, nanocorpuscles with improved stability may be obtained.

In an embodiment, the molar ratio of phospholipid/tensoactive agent ranges between about 0.5 to about 40, preferably between about 2 to about 20, and most preferably between about 3 to about 5.5.

In an embodiment, a Liposomal ALA composition of the present disclosure includes a co-solvent or co-scattering agent. In an embodiment, the co-solvent or co-scattering agent is a pharmaceutically acceptable alcohol. In an embodiment, the pharmaceutically acceptable alcohol is ethanol. Examples of suitable co-solvents may include, but are not limited to, glycols and derivatives, particularly glycerine, propylene glycol, butylene glycol, pentylene glycol and ethoxydiglycol at concentrations between 0.1% and 20%.

In an embodiment, a Liposomal ALA composition of the present disclosure include additives and coadjuvants. In such embodiments, additives and coadjuvants include, but are not limited to, gel formers, membrane stabilizers, membrane preservatives, especially those resulting from para-aminobenzoic acid including methylparaben, propylparaben, butylparaben, isobutylparaben, other organic acids and their salts, such as sorbic acid or benzoic acid and alcohol derivatives can also be used, such as phenoxyethanol.

In an embodiment, a Liposomal ALA composition of the present disclosure includes antioxidants. Without wishing to be bound to any one theory, it is believed that adding antioxidants to a Liposomal ALA composition of the present disclosure may minimize size effects resulting from phototoxic reactions from the reactive oxygen species generated during a PDT treatment. That is, antioxidants may counterbalance side effects from the reactive oxygen species generated during the photochemical reaction triggered by directing appropriate wavelengths to the target tissue containing the protoporphyrin IX. In an embodiment, a Liposomal ALA composition of the present disclosure includes antioxidants to counterbalance side effects from reactive oxygen species generated during a PDT treatment. In an embodiment, antioxidants may minimize harmful side effects of reactive oxygen species in the target tissue. In an embodiment, antioxidants may protect non-target tissue from harmful effects of the reactive oxygen species. In an embodiment, antioxidants may prevent reactive oxygen species generated during a photodynamic therapy treatment from proliferating in non-target tissue. In such embodiments, antioxidants include, but are not limited to, oligopeptides, proteins, salts, buffering substances, hydro soluble active ingredients, vitamins, phloretin, and other similar antioxidants.

In an embodiment, a Liposomal ALA composition of the present disclosure includes phloretin. In such embodiments, the concentration of phloretin ranges between about 0.1% and about 15%. If desired, phloretin can be added to a Liposomal ALA composition of the present disclosure with lipids, surfactants or separately.

In an embodiment, a Liposomal ALA composition of the present disclosure further comprises relative high amounts of linoleic acid and linolenic acid. It is believed that linoleic acid and linolenic acid restore the skin barrier, reduce oil production, and normalize sebum content. Linoleic acid and linolenic acid may help prevent inflammatory reactions frequently observed when using conventional ALA or MALA. In an embodiment, linoleic acid may reduce inflammation induced during a photodynamic therapy treatment of the present disclosure. In an embodiment, linolenic acid may reduce inflammation induced during a photodynamic therapy session of the present disclosure. In an embodiment, including linoleic acid in a Liposomal ALA composition of the present disclosure may minimize a patient's pain or discomfort during a photodynamic therapy treatment of the present disclosure. In an embodiment, linolenic acid may reduce a patient's pain or discomfort during a photodynamic therapy treatment of the present disclosure.

Surprisingly, the compositions of the present disclosure exhibit a high capacity of diffusion of the nanocorpuscles through tissues and a high ability to cross biological barriers (such as the skin barrier and even blood brain barrier). As a result, the ALA compositions of the present disclosure may be used in production of a medicine for the treatment of skin and various medical conditions where an ability to diffuse across biological barriers is desired.

The Liposomal ALA compositions of the present disclosure may be provide significant advantages over conventional ALA products, including, but not limited to: enhanced penetration; prolonged product shelf-life; low dose; decreased side effects; decreased incubation time; and improved delivery. In an embodiment, the Liposomal ALA compositions of the present disclosure may provide enhanced penetration into the skin. In such embodiments, the enhanced penetration into the skin may include penetrating the skin to a depth reaching the hair bulb and subcutaneous fat. In such embodiments, the enhanced penetration into the skin may include penetrating the skin to a depth sufficient to provide a therapeutic effect. In such embodiments, the enhanced penetration into the skin may include penetrating the skin to a depth sufficient to trigger the enzymatic conversion of ALA to protoporphyrin IX. In such embodiments, the enhanced penetration into the skin may include penetrating the skin to a depth sufficient for a PDT treatment to be effective. In such embodiments, the enhanced penetration into the skin may include penetrating the skin to a depth sufficient to generate reactive oxygen species. In such embodiments, the enhanced penetration into the skin may include penetrating the skin to a depth coinciding with a depth of wavelengths directed into the skin.

In an embodiment, the Liposomal ALA compositions of the present disclosure have increased stability. In an embodiment, the increased stability of the Liposomal ALA compositions of the present disclosure may prolong the shelf life of the composition. In an embodiment, the shelf life of the Liposomal ALA compositions of the present disclosure may be as long as about one year and a half. In an embodiment, the prolonged shelf life of the Liposomal ALA compositions, fabrication of ALA encapsulated in nanocorpuscles can be performed for as long as about a year and a half before a treatment using a Liposomal ALA composition. In an embodiment, ALA encapsulated in nanocorpuscles may be fabricated for up to a year before a treatment using the Liposomal ALA compositions of the present disclosure. In an embodiment, the Liposomal ALA compositions of the present disclosure may be fabricated or prepared for as much as six months prior to a treatment using the Liposomal ALA composition. In an embodiment, Liposomal ALA compositions of the present disclosure may be fabricated as much about 6 months, about 12 months, or about 18 months before a PDT treatment using ALA encapsulated in nanocorpuscles.

In an embodiment, the Liposomal ALA compositions of the present disclosure may effectively elicit phototoxic reactions in significantly lower doses than that of conventional ALA. Without wishing to be bound to any one theory, it is believed that the Liposomal ALA compositions of the present disclosure may effectively elicit phototoxic reactions with concentrations ranging from about 0.001 % to about 10%. It is also believed that decreasing the concentration (e.g., dose) of ALA may decrease side effects. In an embodiment, lower doses (e.g., less than about 10%) of ALA mitigate pain during a treatment. In an embodiment, lower doses of ALA mitigate pain during a PDT treatment. In an embodiment, lower doses of ALA mitigate pain following a treatment. In an embodiment, lower doses of ALA mitigate pain following a PDT treatment.

In an embodiment, the Liposomal ALA compositions of the present disclosure include at least one anti-inflammatory to reduce side effects. In an embodiment, the anti-inflammatory comprises phospholipids. In an embodiment, side effects reduced from the Liposomal ALA compositions of the present disclosure include, but are not limited to, swelling, redness, crusts, persistent photosensitivity, and combinations thereof.

In an embodiment, the Liposomal ALA compositions of the present disclosure are believed to improve delivery of the 5-ALA photosensitizer to target tissues. In an embodiment, the improved delivery comprises decreased incubation time. While not wishing to be bound to any one theory, it is believed that 5-ALA encapsulated in nanocorpuscles can reduce the contact time between administration of the 5-ALA and irradiation of the skin because the active 5-ALA is capable of reaching a target, as well as accumulating inside the skin readily within from about 30 minutes to about 90 minutes due to 5-ALAs diffusion rate across membranes. In an embodiment, contact time (e.g., incubation period) may depend on the route of administration. In an embodiment, incubation time comprises from about 15 minutes to about 90 minutes. In an embodiment, the incubation time of 5-ALA encapsulated in nanocorpuscles ranges from about 30 minutes to about 60 minutes. In an embodiment, incubation time ranges from about 30 minutes to about 45 minutes. In an embodiment, incubation time resulting from topical application of the Liposomal ALA compositions of the present disclosure every 5-10 minutes for 6 to 12 times results in incubation times of about 15 minutes to about 90 minutes. In other words, ALA may be converted to protoporphyrin IX within about 15 to about 90 minutes. In an embodiment, intralesional injections can lead to conversion of ALA to protoporphyrin IX within about 15 minutes to about 45 minutes.

In an embodiment, the Liposomal ALA compositions of the present disclosure are believed to improve delivery of the 5-ALA photosensitizer to target tissues. In an embodiment, the improved delivery comprises rapid diffusion. In an embodiment, improved delivery of the Liposomal ALA compositions of the present disclosure comprises specificity for target tissues. In an embodiment, improved delivery of the Liposomal ALA compositions of the present disclosure comprises specificity for tumor cells. In an embodiment, improved delivery of the Liposomal ALA compositions of the present disclosurecomprises high potency.

In an embodiment, improved delivery of the Liposomal ALA compositions of the present disclosure comprises increased availability of routes of administration. In an embodiment, the Liposomal ALA compositions of the present disclosure can be administered without using an occlusion dressing due to the compatibility of the Liposomal ALA composition and the skin conferred by the nanocorpuscles.

The cosmeceutical compositions of the present disclosure can be administered topically (e.g., cutaneous, epicutaneous, transdermal, inhalational, and intranasal), parenterally (intradermal, intraperitoneal, intramuscular, subcutaneous, and intravenous), and via mesotherapy techniques, widening the range of applications, and, more generally, one may use any of the galenic forms that do not, by their particular nature or by their components, impede or improperly reduce the release of the ALA in the area to be treated. In an embodiment, the cosmeceutical compositions of the present disclosure are administered topically. In an embodiment, the cosmeceutical compositions of the present disclosure are administered topically via a spray. In an embodiment, the cosmeceutical compositions of the present disclosure are administered topically via a transdermal patch. In an embodiment, the cosmeceutical compositions of the present disclosure are administered topically via a face/body mask. In an embodiment, the cosmeceutical compositions of the present disclosure are administered via injection. In an embodiment, the cosmeceutical compositions of the present disclosure are administered via subcutaneous injection. In an embodiment, the cosmeceutical compositions of the present disclosure are administered via intravenous injection. In an embodiment, the cosmeceutical compositions of the present disclosure are administered directly to a tumor lesion for treatment of a malignancy. In an embodiment, the cosmeceutical compositions of the present disclosure are administered intravenously for treatment of a malignancy. For example, for a skin cancer, the cosmeceutical compositions of the present disclosure can be injected inside, around or a combination thereof, the tumor. In an embodiment, the cosmeceutical compositions of the present disclosure are administered via mesotherapy with microinjections of the cosmeceutical compositions into subcutaneous fat. Mesotherapy can be performed, for example, via mesotherapy injectors, mesotherapy guns, and mesotherapy injections by hand.

In one aspect, the present disclosure provides a method of preparing an ALA nanocorpuscle that includes: preparing a watery solution of ALA; preparing a solution of phospholipids with tensoactive or its derivatives; homogenizing both solutions as a whole. In an embodiment, after each step and/or at the last step, the method further comprises diluting to add at least an additive, a coadjuvant, a pharmaceutically acceptable excipient and/or their mixtures. Thus, with the method of the present disclosure it is possible to get a Liposomal ALA composition with a concentration of each of the compounds (phospholipid, bile salt or salts, etc.) suitable for administration, or one can obtain a Liposomal ALA composition that would require an additional dilution step (adding, at least, an additive, a coadjuvant, a pharmaceutically acceptable excipient and/or their mixtures) in such a way that a final Liposomal ALA composition suitable for being administered is achieved. In an embodiment, the dilution step is carried out by adding water. In an embodiment, the method further comprises after each step and/or at the lasts step filtering. In an embodiment, the filtering is sterile filtration.

In an embodiment, a Liposomal ALA composition of the present disclosure is fabricated using the following protocol and amounts of agents: approximately 0.17 gram of ALA is dissolved in about 1.35 ml of ethanol and about 11.25 ml of water. Next, approximately 1.53 gram of soy lecithin is mixed in about 0.83 ml of polysorbate 20 with 1.5 ml of ethanol. Both solutions are mixed intensively and the resulting heterogeneous mix is sterilized by filtration at 5 bars of pressure by means of 0.2 microns cellulose filter. The homogeneous dispersion of the obtained liposomes is adjusted to the desired pH and then diluted with bidistillate water until a final concentration of ALA of approximately 0.5%.

Any light source, either laser or nonlaser, with suitable spectral characteristics and a high output at an absorption maximum of the photosensitizer can be used for photodynamic therapy (PDT).

### Lasers

The purpose of lasers in PDT is to initiate photochemical reactions in contradistinction to their photothermal or photomechanical effects, as are seen in other dermatologic uses. Almost any laser with an output wavelength within the visible spectrum (400-800 nm) may be used to activate ALA, although efficiency may be compromised if the output wavelength does not approximate the spectral absorption peak of the photosensitizer. Laser PDT has many advantages, including, but not limited to the monochromaticity of lasers provides maximum effectiveness if the wavelength of the laser corresponds with the peak absorption of the photosensitizer; lasers can produce high irradiance to minimize the therapeutic exposure time; and lasers can be readily coupled to fiberoptics, enabling light delivery to any organ, such as the bladder, gastrointestinal tract, or lungs.

### Noncoherent Light

In the treatment of large skin lesions, noncoherent light sources are superior to laser systems because of their large illumination fields. Additionally, polychromatic light sources allow the use of different photosensitizers with different absorption maxima.

In current dermatologic use, the most widely accepted application of ALA PDT is with blue light for the treatment of AKs. FDA-approved blue light sources include the Blu-U and ClearLight systems. Blue light (410 nm) has a limited skin penetration (less than 0.5 mm). In an embodiment, the nanocorpuscle compositions of the present disclosure merge with the skin barrier layers where the nanocorpuscles release the encapsulated ALA. This leads to an increased local permeability of the barrier layer membranes and the active agents are able to pass through. Therefore, longer wavelengths can be used for activation, such as 550 nm (yellow), 630 nm (red), or a mix of both (orange), which leads to a deeper activation of ALA reaching the skin close to 2 mm or even more. This deeper activation can be important to treat precancerous and cancerous skin tumors or even to induce collagen activation and repair the skin deeply. In an embodiment, a commercial light source, such as TriWings manufactured by Biophoton in France can be used with a PDT treatment of the compositions of the present disclosure. The TriWings light source provides an irradiance higher than 100 mW/cm2, which allows shorter treatment times. Blue light sources can also be used with the compositions of the present disclosure, for example, for superficial conditions such as hyperpigmentation problems (melasma).

In an embodiment, cosmeceutical compositions of the present disclosure can be used in the treatment of a variety of medical conditions, diseases and disorders, as well as dermatological applications, benign and malignant. In an embodiment, cosmeceutical compositions of the present disclosure can be used for the treatment of conditions including, but not limited to, lung cancer, gastrointestinal tract cancers including: esophageal cancer, tumors of the stomach (peritoneal cancer), colorectal cancer, tumors of the small intestine, cancers of the anus, pleura cancer, brain cancer, eye cancer, skin cancer (basocellular carcinoma), atherosclerosis, infectious diseases, rheumatoid arthritis, cutaneous T-cell lymphoma, Kaposi's sarcoma, malignant melanoma, carcinoma in situ (CIS), keratoacanthoma, extramammary Paget's disease, melasma, viral warts, acne vulgaris, vitiligo, lichen sclerosus and atrophicus, sebaceous gland hyperplasia, Psoriasis, hidradenitis suppurativa, actinic keratosis, psoriasis vulgaris, verrucae vulgaris, molluscum contagiosum, actinic chelitis, Hirsutism, alopecia areata, Bowen disease, Stretch marks, enhance healing (e.g., skin healing), skin photorejuvenation/photoaging, and many other medical conditions affecting lungs, brain, esophagus. The cosmeceutical compositions disclosed herein can be used in PDT.

In an embodiment, the cosmeceutical compositions of the present disclosure can be used as a diagnostic aid in, for example, tumoral lesions, where protoporphyrin IX accumulates and may be detected by fluorescent techniques.

### Phloretin - Penetration Enhancer Product of the present disclosure

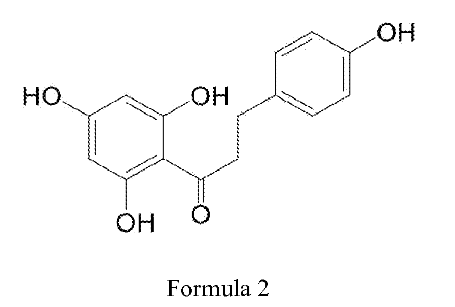

Phloretin (Formula 2) is a flavonoid found exclusively in apples and in apple-derived products where phloretin is present as the glucosidic form, namely, phloridzin (phloretin 2'-*O-*glucose). Phloretin may protect against free radicals and other reactive molecules known to cause damage and DNA mutations among the integral cell types. Phloretin may also correct existing damage by stimulating the synthesis of essential proteins and fibers and accelerating cell turnover. For example, phloretin may inhibit the formation of MMP-1, an enzyme responsible for breaking down collagen. Another example in which phloretin may correct existing damage is by interrupting melanin synthesis to potentially reduce unwanted skin discolorations. Phloretin is also believed to have immunosuppressor, anti-inflammatory, antifungal, and anti-neoplastic properties which may be useful in accomplishing a synergistic effect in the presently disclosed embodiments. Phloretin may also act as penetration enhancer for the percutaneous delivery of certain topically applied drugs by increasing the fluidity of the intercellular lipid bilayers of the stratum corneum. Its mechanism of action is believed to be due to its increase of the fluidity of the intercellular lipid bilayers of the stratum corneum. In this way phloretin will interact also with the liposome and with the natural skin membranes. It is believed that phloretin interacts with stratum corneum lipids to reduce the diffusional resistance of the stratum corneum to drugs and balance the hydrophilic-lipophilic characteristics. In an embodiment, ALA compositions of the present disclosure comprise phloretin encapsulated in nanocorpuscles. In an embodiment, phloretin encapsulated in nanocorpuscles can be used to enhance the epidermal penetration of ALA encapsulated in nanocorpuscles. In an embodiment, phloretin encapsulated in nanocorpuscles can be used to induce an antioxidant reaction to mitigate oxidative stress to the skin resulting from the photochemical reaction. In an embodiment, a cosmeceutical composition of the present disclosure comprises a phloretin product, which is a concentration of phloretin in a mix of ethanol and macrogol.

Although phloretin per se may be used for the presently disclosed embodiments, a person of ordinary skill in the art will appreciate that derivatives of phloretin, e.g., dihydrochalcone derivatives, and other dermatologically effective derivatives of phloretin, may also exhibit similar functionality when used as concentrated solutions in lieu of phloretin, and such functional equivalents of phloretin are intended to be within the spirit and scope of the presently disclosed embodiments.

It will be understood, therefore, that the nanocorpuscles, or a portion of the nanocorpuscles, may comprise a flavonoid, for example phloretin.

In aspects of the present disclosure, cofactors may be used to decrease side effects of PDT and enhance release of the ALA encapsulated in nanocorpuscles once the ALA has penetrated the skin. Cofactors may also be used in aspects of the present disclosure to stimulate the transformation of ALA into protoporphyrin IX. Without wishing to be bound to any one theory, it is believed that cofactors may enhance the conversion of ALA to protoporphyrin IX by activating the pathway from ALA to HEME thereby improving a PDT treatment.

### Iron Chelates - Chelating Products of the present disclosure

The rate-limiting enzyme responsible for the synthesis of ALA from the amino acid glycine and succinyl-CoA is ALA synthase. ALA synthase is regulated by, among others, intracellular iron levels. Low levels of intracellular iron therefore may lead to decreased porphyrin synthesis. Without wishing to be bound to any one theory, it is believed that iron chelates can be used as cofactors to sequester iron in a manner that catalyzes the enzymatic conversion of ALA to porphyrin IX. In an embodiment, a cofactor of the present disclosure comprise iron chelates. In an embodiment, the iron chelate comprises zinc salts encapsulated in nanocorpuscles. In an embodiment, the iron chelating zinc salt includes, but is not limited to, zinc chloride, zinc gluconate, or zinc acetate. In an embodiment, the iron chelate comprises copper salts encapsulated in nanocorpuscles. In an embodiment, the iron chelating copper salt includes, but is not limited to, copper gluconate or copper acetate. In an embodiment, the iron chelate comprises a mix of zinc and copper or zinc salts or copper salts encapsulated in nanocorpuscles. In an embodiment, the iron chelate comprises lactoferrin-peroxidase encapsulated in nanocorpuscles. In an embodiment, the iron chelate comprises a mix of zinc chloride, copper, and lactoferrin-lactoperoxidase encapsulated in nanocorpuscles. In an embodiment, the iron chelate can sequester iron to catalyze the enzymatic conversion of 5-aminolevulinic acid to the porphyrin.

As the iron chelates or metal chelates may be mentioned sources of iron-complexing agents or, respectively, sources of metal complexing agent. Thus the aforesaid zinc and copper salts act as a source of iron-complexing agents, as does lactoferrin-peroxidase. Alternatively stated, it is contemplated in embodiments that the metal/iron chelates may be described as metal/iron sequestering agents which remove metal/iron from free solution.

The nanocorpuscles, or a portion thereof, may therefore comprise a source of metal-complexing agent and in particular a source of iron-complexing agent.

### OxySeS

In an embodiment, OxySeS is a mist (spray) provided comprising oxygen saturated liposomes, and 10 mg/ml of fructose 1, 6 bisphosphate encapsulated in unilamellar liposomes. In an embodiment, OxySes is referred to as an "oxygen product" of the present disclosure. In an embodiment, OxySeS includes one or more of the following ingredients: water, lecithin, alcohol denat (e.g., denaturated alcohol), fructose, tocopherol, sodium chloride, sodium hydroxide, sodium cholate, and phenoxyethanol. In an embodiment, OxySeS comprises a spray formulation. In an embodiment, OxySeS comprises a foam formulation.

OxySeS increases the oxygen content of the skin by delivering O₂ through unilamellar liposomes. It is believed that the fibroblast cell activity, responsible for manufacturing collagen and elastin, is dependent on the availability of O₂. Fructose 1, 6 bisphosphate is provided for anti-inflammatory and antiradicalar action. Fructose 1, 6 bisphosphate also may promote skin repair and may be useful for conditions associated with poor blood supply or tissular injury. In an embodiment, a main component of the liposomes is phosphatidylcholine (PC). Phosphatidylcholine may be provided: for repairing the stratum corneum to improve the barrier function of skin; for anti-inflammatory action to reduce edema in the treatment area; and for bactericidal activity to avoid proliferation of post-treatment infection.

In an embodiment, OxySeS is applied to the skin to supply additional oxygen to skin tissue before applying a cosmecuetical composition of the present disclosure.

OxySeS may be effective in cases in which an increase of tissue oxygenation is desired, such as photodynamic therapy. Indications for OxySeS therefore may include plastic surgery, phlebology, diabetes, dermatology, and radiotherapy. OxySeS may be indicated for use in plastic surgery following phenol or TCA peels, skin grafts, and for speeding healing processes. In the area of phlebology, OxySeS may be particularly useful for treating venous ulcers and skin necrosis related to sclerotherapy. In the care of diabetes, OxySeS may help diabetics prevent keloid formation. OxySeS may also ameliorate various skin conditions associated with diabetes, such as diabetic feet, diabetic ulcers, and may improve skin elasticity. Dermatological uses of OxySeS can include improving skin elasticity, use before, during, or after aesthetic surgery, pre-peel treatment or post-peel treatment, microdermabrasion, or generally for conditions caused by poor blood supply, smoking, or anaerobic skin infections. Radiotherapy application of OxySeS may include radiodermatitis treatment, skin necrosis, or the like.

In an embodiment, prior to applying a cosmecuetical composition of the present disclosure to the skin, it may be beneficial to apply OxySeS to the areas of skin to be treated on as many as seven or fourteen days prior to treatment for optimal results. In those instances where a patient to be treated has impaired circulation, OxySeS may be applied on the area to be treated. Application twice a day for up to as much as about four weeks before the procedure may be advisable. However, less frequent daily application as early as a day before the procedure may also be helpful.

Post procedure use of OxySeS may include application of OxySeS immediately after irradiation of the treated area by gentle rubbing. In an embodiment, post procedure use of OxySeS may include applying OxySeS twice daily for a period of up to about four weeks.

Without wishing to be bound to any one theory, it is believed that the availability of O₂ enhances photodynamic therapy. That is, increased supply of O₂ to target tissue during a PDT treatment can improve the outcome of the PDT. It is believed that overcoming the depletion of O₂ can enhance the results of a PDT treatment. In an embodiment, O₂ depletion can be overcome by lowering the light fluence rate. In an embodiment, pulse light delivery may allow re-oxygenation of the target tissue.

In an embodiment, OxySeS comprises liposomal fructose 2, 6-bisphosphate in combination with oxygen saturated liposomes to facilitate activation of the ALA pathway to heme. In an embodiment, using OxySeS in conjunction with iron chelates of the present disclosure may confer a synergistic effect to enhance the enzymatic conversion of ALA to protoporphyrin IX.

In an embodiment, the present disclosure provides respective kit assemblies or "treatment kits" for treating the respective skin conditions set forth above. In an embodiment, each respective kit assembly is prepackaged to contain the appropriate topical agents and supplies, and includes an effective and convenient instructional means, such as an instructional pamphlet or a videotape or other instructional means containing thereon indicia for administration of sequentially applied products according to steps needed for the respective skin condition to be treated.

In an embodiment, the kit assembly also provides a sequential dispenser means containing a plurality of daily sets of kit sub-assembly components, such as a series of jars, bottles, containers or ampoules containing a supply (unit dose) of chemical solution. In addition to respective kits being specific for treatment of each aforementioned skin condition, kits are further respectively specific with regard to whether a given kit is to be used for the therapeutic phase or, in the alternative, for a maintenance phase.

Generally, and except as set forth for specific skin conditions, each kit provided in the present disclosure has sub-assembly components including therein the following: a unit dose of a photosensitizer composition, the photosensitizer composition comprising nanocorpuscles comprising the photosensitizer 5-aminolevulinic acid in a scattering agent; a unit dose of a penetration enhancer product, the penetration enhancer product comprising nanocorpuscles comprising a flavonoid in a solution; a unit dose of a chelating product, the chelating product comprising nanocorpuscles comprising a metal chelate in a solution; and a unit dose of an oxygen product, the oxygen product comprising 100% oxygen saturated liposomes and 10 mg/ml of fructose 1, 6 bisphosphate encapsulated in unilamellar liposomes.

The following Examples are provided to aid in the understanding of the present disclosure and are not construed as a limitation thereof.

### EXAMPLES

### Example 1

Low strength ALA of the present disclosure improves photoaging signs when applied several times in a short period of time (about 30-60 minutes), followed by subsequent exposure of the skin to an appropriate light source.

In an embodiment, improved photoaging sings include hyperpigmentation ((dyschromia-improves pigmented and hypopigmented lesions), wrinkles (collagen remodeling) and fine lines), decrease pore size and acne blemishes, improves skin roughness in the face (leads to smoother skin), neck and V of the shoulder., improves yellowish color, increase skin thickness & dyschromia).

A Liposomal ALA composition disclosed herein can be formulated as a nano-spray. In an embodiment, the Liposomal ALA nano-spray comprises ALA encapsulated in liposomes as a photo mist. The Liposomal ALA nano-spray has enhanced penetration, activity, and efficacy inside the skin. The Liposomal ALA nano-spray allows for a reduced incubation period (as compared to topical compositions currently on the market). Incubation time can be reduced to a range from about 30 minutes to about 60 minutes. In an embodiment, the Liposomal ALA nano-spray comprises ALA at a reduced concentration (from about 1% to about 2.5%) (as compared to topical compositions currently on the market). In an embodiment, the Liposomal ALA nano-spray can be used for treating moderate wrinkles in phototypes I to V, and sebum reduction/Acne blemishes. In an embodiment, the Liposomal ALA nano-spray can be used to smooth wrinkles (collagen remodeling) and fine lines, improve roughness in the face, neck and V of the shoulder, improve hyperpigmentation - Dyschromia, improve oily skin and acne blemishes, and decrease pore size.

In an embodiment, a method of using a Liposomal ALA nano-spray of the present disclosure includes: spraying the skin to be treated with the Liposomal ALA nano-spray approximately every five minutes, six to twelve times (about 30-60 minutes), wherein the spraying causes a sufficient amount of ALA to be converted to Protoporphyrin IX; and activating the Protoporphyrin IX, wherein the Protoporphyrin IX is activated by exposing the treated skin to visible light covering the Soret band (407 nm) or Q bands (505, 540, 580 and 635 nm) of Protoporphyrin IX. Any light source emitting between 400-720 nm may be used to activate Protoporphyrin IX. In an embodiment, the light amount is higher than 10 /JCm². Omnilux PDT operates at a fluence rate of 80 mW/cm². In an embodiment, a recommended exposure time is about 20 minutes. Treatments may be repeated every 3-4 weeks in photo rejuvenation type 1. In an embodiment, the method further comprises evaluating the concentration of Protoporphyrin IX in a dermal layer of the skin by measuring the intensity of the obtained fluorescence indirectly with Wood's light.

Once ALA penetrates the skin, ALA will be transformed in the dermal cells to Protoporphyrin IX. A characteristic of Protoporphyrin IX is yellowish-red fluorescence when the skin is exposed to blue (410 nm) and red (633 nm) light, a property which ALA does not possess. Therefore the actual concentration of Protoporphyrin IX in the dermis can be evaluated by measuring the intensity of the obtained fluorescence indirectly with Wood's light.

In previous clinical studies, it was found that significant fluorescence can be obtained after spraying the skin every 5-10 minutes for 30-60 minutes. The fluorescence begins to diminish approximately 15 minutes after finishing the application, indicating less photosensitivity, but the spraying also should be continued up to 5 minutes before the skin exposure to the selected light, independent from the 1 hour lapsed spraying time.

In an embodiment, a treatment procedure for using a Liposomal ALA nano-spray of the present disclosure on a patient includes: examining a skin surface of the patient for signs of carcinoma, wherein the presence of carcinoma would cause the procedure to stop; washing the patient's face and de-greasing the patient's skin; optionally performing a Salicylic acid peel (SaliPeel) or microdermabrasion procedure; washing the patient's face; spraying the Liposomal ALA nano-spray to the patient's entire face (including lids and around lips) every 5 minutes for 30-60 minutes; massaging the patient's skin after each application; repeating the spraying as necessary up to 5 minutes before exposure to a light, even if the spray has been applied for one hour; and exposing the patient's face to an appropriate wavelength of light (400-720 nm). The procedure can further include taking pictures to document the treatment.

In an embodiment, in order for the patient and operator to be properly protected from exposure to the Intense Pulsed Light (IPL) device, the following conditions should be maintained: selecting the appropriate wavelengths (400-720); protecting the patient's and the operator's eyes; applying transmission gel (Ioson IPL, Ioson NAD); carrying out the treatment with the selected hand piece (400-720 nm) using a unique pulse of 3.5 to 5.0 J/cm2 and a pulse duration of 30 to 50 msg; making 3 full face passes (80-1000 shots) or step until adding 10.5 to 15 J/cm², wherein the three passes can be made first on the right half of the face and then on the other half of the face, and wherein the upper and lower lids of the patient are treated with care by dragging the skin to a bony area (a gauze can be used to make a good clip in the skin); avoiding direct light to the eyes; treating less sensitive areas first; and overlapping treatment on areas by about 1 mm.

In an embodiment, a post-treatment care includes removing the transmission gel and washing the patient's face with soap or with cleaning towelettes; applying sunscreen (for example, ScreenSeS 80 colored cream); and advising the patient to avoid sun exposure for a minimum of 12 hours after the treatment and to use a sunscreen for the next 30 days.

### Example 2

Nanocorpuscles comprising 5-aminolevulinic acid in a scattering agent can be used for the preparation of a medicament for the treatment of moderate acne vulgaris by photodynamic therapy (PDT). A Liposomal ALA composition of the present disclosure, having a concentration of about 1% ALA was tested for PDT treatment of moderate acne vulgaris on 24 patients. The Liposomal ALA composition was formulated as a mist, and was applied topically to acne lesions approximately every 5 minutes for about 80 minutes. The lesions were irradiated by a LED light having wavelength of 633 nm at dose levels of 50-70 J/cm² at 66 mW/cm². Substantial reduction of the number and sizes of lesions for majority of patients within 3 courses, with many showing improvement after only one or two treatments. Minimal adverse effects were noted included facial erythema and flaking occurring twenty-four hours after irradiation that lasted between two and four days. This study suggests that 1% liposomal ALA PDT is a safe and effective therapeutic option for the treatment of moderate acne.

### Example 3

Nanocorpuscles comprising 5-aminolevulinic acid in a scattering agent can be used for the preparation of a medicament for the treatment of photoaging of the skin by photodynamic therapy (PDT). Eight patients with light to moderate photoaging signs on the face, neck, and hands, including fine wrinkles, skin thinning, hyperpigmentations, dyschromia (solar lentigines, freckles), erytrosis, and telangiectasias, without evidence of solar keratoses were treated three times spaced apart every three to four weeks with Liposomal ALA compositions of the present disclosure. Two patients were irradiated with Intense Pulsed Light (IPL) (Quanta, eterna Giovienezza). Half of the patients were treated with an about 0.5% liposomal ALA composition of the present disclosure and the other half with an about 1% liposomal ALA composition of the present disclosure. Significantly, hyperpigmented lesions were attenuated in 90% of the cases by the end of the third treatment, more than half by 50% and 25% after only a single treatment. Cutaneous hyperemias and small telangiectasias diminished in a majority of the patients after the third treatment.

### Example 4

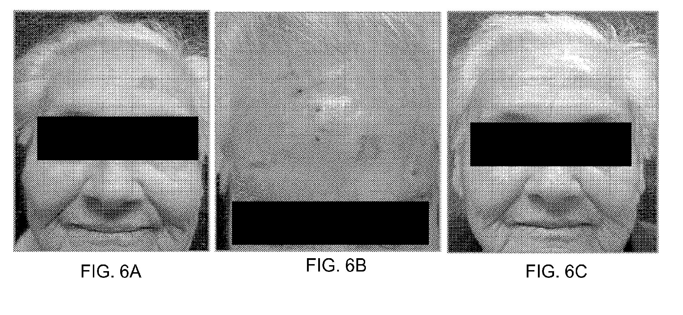

**FIG. 6A, FIG. 6B** and **FIG. 6C** shows use of nanocorpuscles comprising 5-aminolevulinic acid for the manufacture of a photosensitizer for use in photodynamic therapy treatment of photoaging of the skin. In an embodiment, a Liposomal ALA composition of the present disclosure is useful for treating symptoms associated with photoaging of the skin due to exposure to solar radiation. **FIG. 6A** shows a patient with sun damage and solar keratoses on the forehead. **FIG. 6B** shows the patient in **FIG. 6A** with a phototoxic reaction. **FIG. 6C** shows the patient in **FIG. 6A** and **FIG. 6B** after a single Liposomal ALA PDT treatment. As shown in **FIG. 6C**, the symptoms of sun damage and solar keratoses have noticeably cleared after a single Liposomal ALA PDT treatment using a Liposomal ALA composition of the present disclosure.

### Example 5

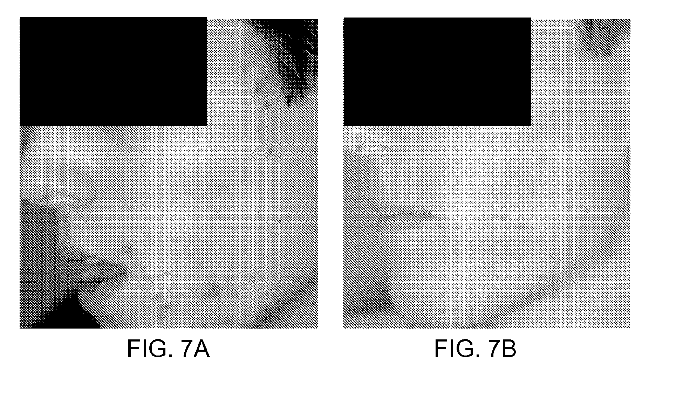

**FIG. 7A** and **FIG. 7B** shows use of nanocorpuscles comprising 5-aminolevulinic acid for the manufacture of a photosensitizer for use in photodynamic therapy treatment of acne vulgaris. In an embodiment, a Liposomal ALA composition of the present disclosure is useful for treating patients with inflammatory acne. **FIG. 7A** shows a patient with inflammatory acne on the patient's cheek. **FIG. 7B** shows the patient in **FIG. 7A** after four treatments at one month intervals. As noted in **FIG. 7B**, the patient's inflammatory acne condition has improved and the patient's skin condition has begun to smooth and clear.

A photodynamic therapy system includes nanocorpuscles comprising 5-aminolevulinic acid in a scattering agent, wherein a concentration of the 5-aminolevulinic acid is from about 0.01% to about 10%; and a light source for activating the 5-aminolevulinic acid.

A kit for photodynamic therapy includes a unit dose of a photosensitizer composition, the photosensitizer composition comprising nanocorpuscles comprising the photosensitizer 5-aminolevulinic acid in a scattering agent; a unit dose of a penetration enhancer product, the penetration enhancer product comprising nanocorpuscles comprising a flavonoid in a solution; a unit dose of a chelating product, the chelating product comprising nanocorpuscles comprising a metal chelate in a solution; and a unit dose of an oxygen product, the oxygen product comprising oxygen saturated liposomes and 10 mg/ml of fructose 1, 6 bisphosphate encapsulated in unilamellar liposomes.

A method for photodynamic therapy includes administering a photosensitizing composition, administering a penetration enhancer for enhancing penetration of the photosensitizing composition into a target tissue, administering a coactivator for catalyzing an enzymatic reaction, and administering a photodynamic therapy treatment. In an embodiment, the photosensitizing composition comprises ALA encapsulated in nanocorpuscles. In an embodiment, the photosensitizing composition is converted via the enzymatic reaction to protoporphyrin IX. In an embodiment, the penetration enhancer comprises phloretin. In an embodiment, the coactivator comprises one of a mist comprising oxygen saturated liposomes and fructose 1, 6 bisphosphate encapsulated in liposomes to increase the oxygen content to the target tissue or a formulation comprising lactoferrin-lactoperoxidase, zinc, copper, nicotinamide to catalyst the conversion of ALA to protoporphyrin IX.

A photodynamic therapy method includes administering nanocorpuscles comprising 5-aminolevulinic acid to a target tissue in a patient; and activating the 5-aminolevulinic acid with a light source.

Nanocorpuscles comprising 5-aminolevulinic acid in a scattering agent can be used for the preparation of a medicament for the treatment of acne vulgaris by photodynamic therapy (PDT). Nanocorpuscles comprising 5-aminolevulinic acid in a scattering agent can be used for the preparation of a medicament for the treatment of photoaging of the skin by photodynamic therapy (PDT).

Nanocorpuscles comprising 5-aminolevulinic acid can be used in the manufacture of a photosensitizer for use in photodynamic therapy treatment of acne vulgaris. Nanocorpuscles comprising 5-aminolevulinic acid can be used in the manufacture of a photosensitizer for use in photodynamic therapy treatment of photoaging of the skin.

All patents, patent applications, and published references cited herein are hereby incorporated by reference in their entirety. It will be appreciated that various of the above-disclosed and other features and functions, or alternatives thereof, may be desirably combined into many other different systems or applications. Various presently unforeseen or unanticipated alternatives, modifications, variations, or improvements therein may be subsequently made by those skilled in the art which are also intended to be encompassed by the following claims.

## Claims

1. A photodynamic therapy system comprising: a composition comprising nanocorpuscles comprising 5-aminolevulinic acid in a scattering agent; and a light source for activating the 5-aminolevulinic acid.

2. The system of claim 1, wherein the nanocorpuscles comprise at least one lipid bilayer, the lipid bilayer comprising at least one phospholipid and at least one tensoactive agent or derivatives thereof.

3. The system of claim 1 or claim 2, wherein the scattering agent is water.

4. The system of any preceding claim, further comprising nanocorpuscles comprising a flavonoid, optionally wherein the flavonoid is phloretin.

5. The system of any preceding claim, further comprising nanocorpuscles comprising a metal chelate.

6. The system of claim 6, wherein the metal chelate is an iron chelate, optionally wherein the iron chelate is an iron-chelating zinc salt, e.g. selected from zinc chloride, zince gluconate or zinc acetate or a combination thereof, or is an iron-chelating copper salt, e.g. copper gluconate or copper acetate, or a combination thereof, or is a combination of such zinc and copper salts.

7. The system of any preceding claim, wherein an output wavelength of the light source is in the visible spectrum.

8. A kit for photodynamic therapy comprising:
a unit dose of a photosensitizer composition, the photosensitizer composition comprising nanocorpuscles comprising 5-aminolevulinic acid in a scattering agent;
a unit dose of a penetration enhancer product, the penetration enhancer product comprising nanocorpuscles comprising a flavonoid in a solution;
a unit dose of a chelating product composition the chelating product comprising nanocorpuscles comprising a metal chelate in a solution; and
a unit dose of an oxygen product, the oxygen product comprising oxygen saturated liposomes and 10 mg/ml of fructose 1, 6 bisphosphate encapsulated in unilamellar liposomes.

9. The kit of claim 8, wherein the nanocorpuscles further comprise one or both of a flavonoid, e.g. phloretin, and an iron chelate, optionally wherein the iron chelate is an iron-chelating zinc salt, e.g. selected from zinc chloride, zinc gluconate or zinc acetate, or a combination thereof, or is an iron-chelating copper salt, e.g. copper gluconate or copper acetate, or a combination thereof, or is a combination of such zinc and copper salts.

10. A composition comprising nanocorpuscles comprising 5-aminolevulinic acid, optionally in a scattering agent, for therapeutic use, and optionally for use in the treatment of acne vulgaris by photodynamic therapy or for use in the treatment of photoaging of the skin by photodynamic therapy.

11. The composition of claim 10 which further includes one or more of the following features:
(i) the scattering agent is water;
(ii) the nanocorpuscles comprise, e.g. consist of, nanocorpuscles comprising a flavonoid, optionally wherein the flavonoid is phloretin;
(iii) the nanocorpuscles comprise, e.g. consist of, nanocorpuscles comprising an iron chelate, optionally wherein the iron chelate is as recited in the optional part of claim 6.

12. The composition of claim 10 or claim 11, which is for use in treating a condition selected from lung cancer, gastrointestinal tract cancers, esophageal cancer, peritoneal cancer, colorectal cancer, tumors of the small intestine, cancers of the anus, pleura cancer, brain cancer, eye cancer, skin cancer, atherosclerosis, infectious diseases, rheumatoid arthritis, cutaneous t-cell lymphoma, Kaposi's sarcoma, malignant melanoma, carcinoma in situ, keratoacanthoma, extramammary paget's disease, melasma, viral warts, acne vulgaris, vitiligo, lichen sclerosus and atrophicus, sebaceous gland hyperplasia, Psoriasis, hidradenitis suppurativa, actinic keratosis, psoriasis vulgaris, verrucae vulgaris, molluscum contagiosum, actinic chelitis, Hirsutism, alopecia areata, bowen disease, stretch marks, skin damage, skin photoaging, and combinations thereof.

13. A method for photodynamic treatment to improve the appearance of the skin comprising:
administering nanocorpuscles comprising 5-aminolevulinic acid to the skin of a subject;
and
activating the 5-aminolevulinic acid with a light source, optionally wherein the nanocorpuscles are formulated in a scattering agent and/or are as defined in claim 11.

14. The subject matter of any preceding claim wherein the nanocorpuscles comprise at least one lipid bilayer, the lipid bilayer comprising at least one phospholipid, e.g. phosphatidyl chlorine, and at least one tensoactive agent, optionally wherein the tensoactive agent (e.g. detergent or surjactant) is a bile salt.

15. The subject matter of any preceding claim wherein 5-aminolevulinic acid is in an amount of from 0.01% to 10%, the percentage being weight %/volume of the composition comprising the nanocorpuscles, and optionally is in an amount of from 0.01% to 5%, e.g. 0.1% to 10%, 0.1% to 5% or 0.1% to 4%, for example 0.5% to 10%, 0.5% to 5% or 0.5% to 4%, as in the case of 2.5% to 10% or 2.5% to 5%.
